# EUROPEAN PATENT SPECIFICATION

(11) **EP 0 883 617 B1**
(45) Date of publication and mention of the grant of the patent: **04.07.2001**
(21) Application number: 96903265.5
(22) Date of filing: 27.02.1996
(51) Int. Cl.: C07D 401/12, C07D 239/69, C07D 213/71, C07C 311/29, A01N 47/36

(54) **HERBICIDAL SULFONAMIDE DERIVATIVES**
HERBIZIDE SULFONAMID-DERIVATE
DERIVES HERBICIDES DE SULFONAMIDE

(43) Date of publication of application: 16.12.1998
(73) Proprietor: LG Chemical Co., Ltd., Daejon 305-380 (KR)
(72) Inventor: KIM, Dae, Whang, Yusung-ku,Daejeon 305-340 (KR); CHANG, Hae, Sung, Yusung-ku,Daejeon 305-333 (KR); KO, Young, Kwan, Yusung-ku,Daejeon 305-333 (KR); RYU, Jae, Wook, Yusung-ku,Daejeon 305-333 (KR); HONG, Sung, Yeap, Daejeon 305-343 (KR); WOO, Jae, Chun, Seo-ku,Daejeon 302-162 (KR); KU, Dong, Whan, Yusung-ku,Daejeon 305-333 (KR); HWANG, In, Taek, Daejeon 302-182 (KR)
(74) Representative: Weber, Dieter, Dr.
(86) International application number: KR9600029
(87) International publication number: WO9731913

(56) References cited:
- WO-A-92/14728
- WO-A-92/15568
- CHEMICAL ABSTRACTS, Vol. 118, No. 15, 12 April 1993, (Columbus, Ohio, USA), page 338, Abstract No. 142346w, AHAN, TAE WOO et al., "Inhibition of Acetohydroxyacid Synthase by Sulfonylureas and Imidazolinones"; & HAN'GUK SAENGHWA HAKHOECHI, 1992, 25(7), 636-41.
- CHEMICAL ABSTRACTS, Vol. 124, No. 15, 08 April 1996, (Columbus, Ohio, USA), page 405, Abstract No. 196428s, SUNG, NACK-DO et al., "Herbicidal Activity and Persistency in Aqueous Solution of Ortho-Disubstituted Benzenesulfonylurea Derivatives"; & HAN'GUK NONGHWA HAKHOECHI, 1995, 38(6), 570-6.

## Description

### BACKGROUND OF THE INVENTION

### Field of the Invention

The present invention relates to novel herbicidal sulfonamide derivatives of the following formula (I) having erythro stereochemistry as herbicides for treatment of pre-emergence and/or post-emergence, their use and composition as agriculturally suitable herbicides. 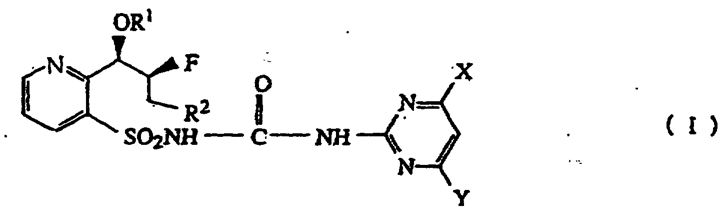 wherein,
- R¹: is 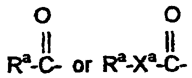 group, wherein R^{a} is C₁-C₃ alkyl, C₁-C₃ haloalkyl, C₂-C₃ alkenyl or C₂-C₃ alkynyl group, wherein X^{a} is O, S, NH or NR^{a} group;
- R²: is C₁-C₂ alkyl group; and
- X and Y: are independently halogen atom, C₁-C₂ alkyl, C₁-C₂ alkoxy or C₁-C₂ haloalkoxy group.

### Description of the Prior Art

It is publicly well-known that sulfonyl urea derivatives possess a herbicidal activity. Such examples containing sulfonyl urea are;
(1) Korea Patent No. 70,675 discloses the compound having the following formula(A) 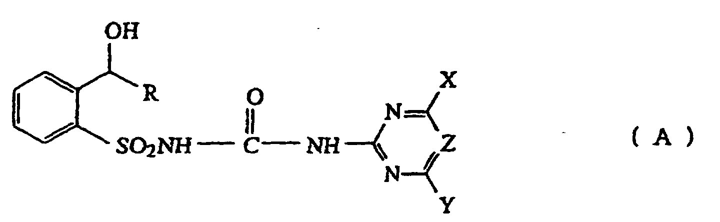 wherein,
   R is haloalkyl group;
   X and Y are independently CH₃, OCH₃ or Cl etc.;
   Z is CH or N.
(2) Korea Patent No. 70,677 and WO 9214728 disclose the compound having the following formula (B) 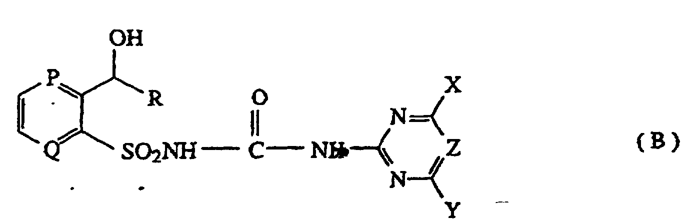 wherein,
   R, X, Y and Z are as previously defined,
   P and Q are differently N or CH, and present as pyridine ring.

If R group of the above formula(A) and (B) includes asymmetric carbon atom, then the above compounds have two stereoisomers which are threo and erythro stereoisomer by reason of two asymmetric carbon atoms. But the above stereoisomers are different each other in herbicidal activity and selectivity.

### SUMMARY OF THE INVENTION

The object of the present invention is to provide novel sulfonamide derivatives having a good selectivity toward rice and also possessing very prominent herbicidal activities for annual and perennial weed, especially a barnyard grass.

Another object of this invention is to provide herbicidal compositons containing said sulfonamide derivatives as active compounds.

### DETAILED DESCRIPTION OF THIS INVENTION

The present invention relates to herbicidal sulfonamide derivatives of the following formula(I) having erythro stereochemistry, which have herbicidal selectivity toward rice, and their agriculturally suitable salts. 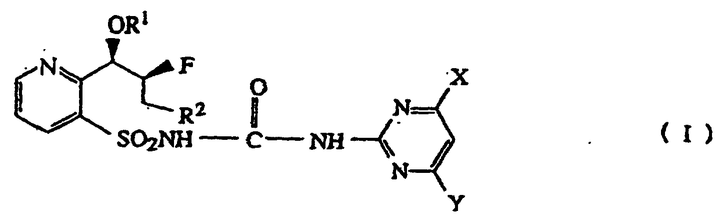 wherein,
P, Q, R¹, R², X and Y are as previously defined.

Preferred compounds having erythro stereochemistry of the above formula (I), in view of a strong activity and a good selectivity are compounds wherein R¹ is acetyl group and X and Y are respectively methoxy group.

These compounds can easily control barnyard grass as well as a perennial weed causing trouble for rice and can be used agriculturally as herbicidal composition for rice. Especially the following compounds have a good selectivity for rice:
Erythro-2-(1-acetoxy-2-fluoro-*n*-butyl)-*N*-[(4,6-dimethoxypyrimidin-2-yl) aminocarbonyl]-3-pyridinesulfonamide[compound No. 1].

The erythro stereoisomer of the above formula(I) according to the present invention has more prominent herbicidal activity than threo stereoisomer or mixture of erythro and threo stereoisomer.

Furthermore, the erythro stereoisomer of the above formula(I) may be used as herbicides or active ingredient of herbicidal composition because of a good selectivity for rice.

A pure compound having erythro stereochemistry of the above formula(I) according to the present invention can be prepared by reactions described in herein below, but should not be constructed to be limited hereto.

Also, the compounds of the above formula (I) according to the present invention can be prepared by reacting the erythro stereoisomer having the following formula (II) with the compound having the following formula (III). 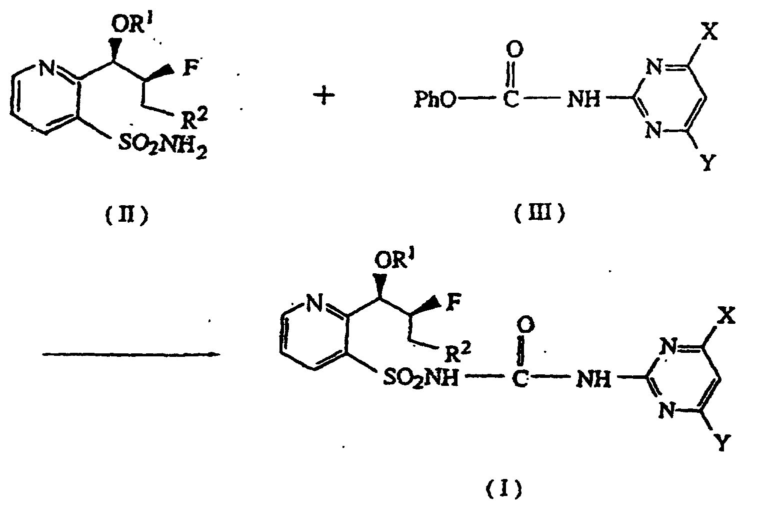 wherein,
R¹, R², X and Y are respectively defined as the above formula(I).

In the above reaction, unreacting solvent such as tetrahydrofuran, acetone, acetonitrile, dioxane, methylene chloride, toluene, butanone, pyridine, dimethylformamide, etc., may be used.

The reaction may be preferably carried out under strong base such as DBU or DABCO, etc. in a small quantity at the temperature of 20~80°C. The above reaction is referred to in U.S. patent No. 4,443,245 and thereafter the desired product can be obtained by acidifying by the method mentioned in European Patent No. 44,807. If necessery, a pure product can be obtained by purification by HPLC. Said, DBU represents 1,8-diazabicyclo[5.4.0] undec-7-ene, and DABCO represents 1,4-diazabicyclo [2.2.2]octane.

Also, the compounds of the formula(III) used for preparing the above formula(I) can be easily obtained by the prior art.

On the other hand, the erythro stereoisomer of the above formula(II) can be prepared by the following reaction scheme. 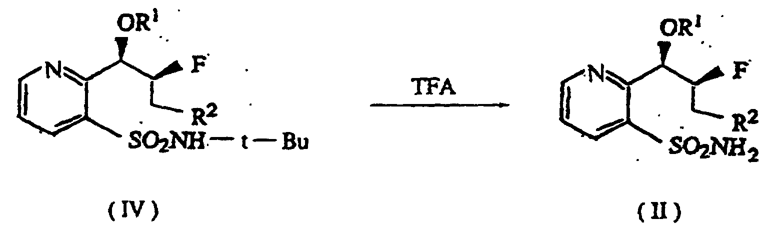 wherein,
R¹ and R² are respectively defined as the above.

In the above reaction, the primary sulfonamide having erythro stereochemistry of the above formula(II) can be prepared by treating *N-t*-butylsulfonamide of the above formula(IV) with an acid such as trifluoroacetic add (TFA) at the temperature of 0~50°C.

Also, the erythro stereoisomer of the above formula(IV) used in the above reaction can be prepared by common acylation of the following formula(V). The pure erythro stereoisomer of the above formula(IV) can be obtained from a mixture of threo and erythro stereoisomer by separation method such as column chromatograph, HPLC or prep-TLC.

The compounds of the following formula(V) can be prepared by selective reduction of the compound of the following formula(VI) with selective reducing agent such as diisobutylaluminum hydride. 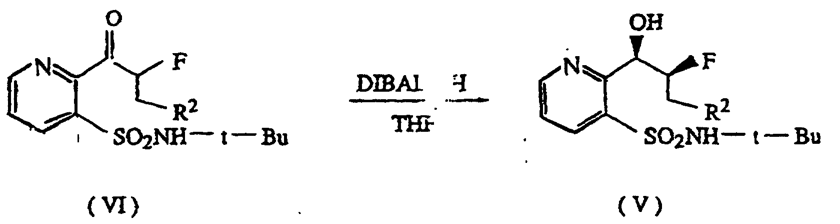 wherein,
R² is defined as the above,
DIBAL • H is diisobutylaluminum hydride.

The pure erythro stereoisomer of the above formula(V) can be easily purified using column chromatograph.

The compound of the above formula(IV) can also be prepared by another process as shown in the following reaction. 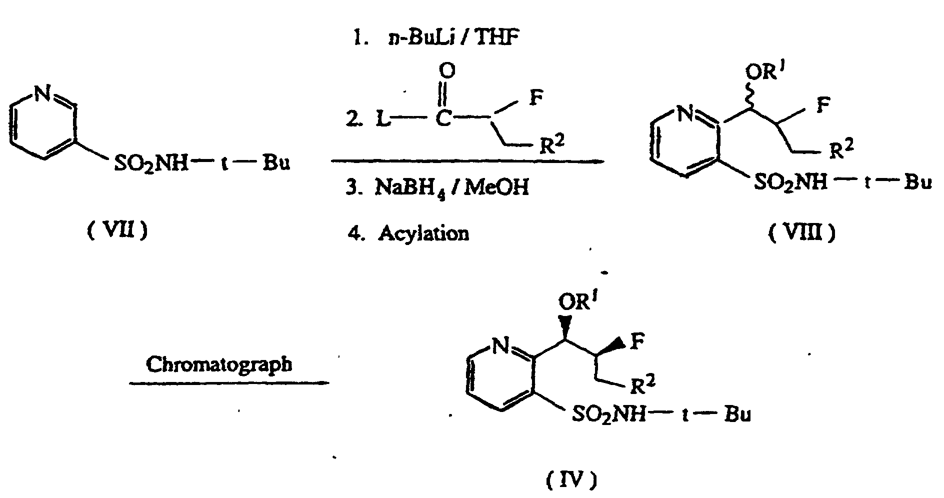 wherein,
R² is defined as the above formula (I),
R¹ is defined as the above formula(I),
L is alkoxy, N(CH₃)₂ or NCH₃(OCH₃), etc.

The above reaction process has been disclosed in Korea Patent No. 70,675 and No. 70,677. *n*-Butyl lithium of 2 equivalents are added in the compound of the above formula(VII) in THF solvent for 1~24 hours at -100 ∼ +30°C to obtain dilithio salt, and then 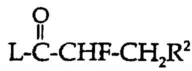 is added at -100 ∼ -40 °C to obtain ketone compound. Hydroxy compound is obtained by reduction of the above ketone compound with NaBH₄, and then the compound of formula (VIII) wherein R¹ is acetyl group is obtained by acylation under acetic anhydride, DMAP and pyridine.

The pure erythro stereoisomer of the above formula (IV) can be easily obtained by separation and purification techniques such as HPLC, column chromatograph, prep-TLC, etc..

On the other hand, salts of the compound of the above formula(I) which are also useful as herbicide, can be prepared by various methods according to prior art. For example, metal salts of the compound can be prepared by reacting the above formula(I) compound with strong basic anion, e.g. alkali or alkaline earth metal solution having hydroxyl group, alkoxide or carbonate, and also quaternary amine salt alike.

A salt of the formula(I) compound may also be obtained by cation exchange. The cation exchange can be carried out by directly reacting a solution containing cation for exchange with the solution of salt of formula(I), for example aqueous solution of alkali metal or quaternary amine salt. This method is useful when the desirable salt is water soluble, especially sodium, potassium or calcium salt.

The above manufacturing methods are summarized briefly, and the methods can be carried out easily by a person skilled in the technical field for manufacturing sulfonyl urea or organic composition.

The compounds of the above formula(I) according to the present invention may be specified as the following Table 1.

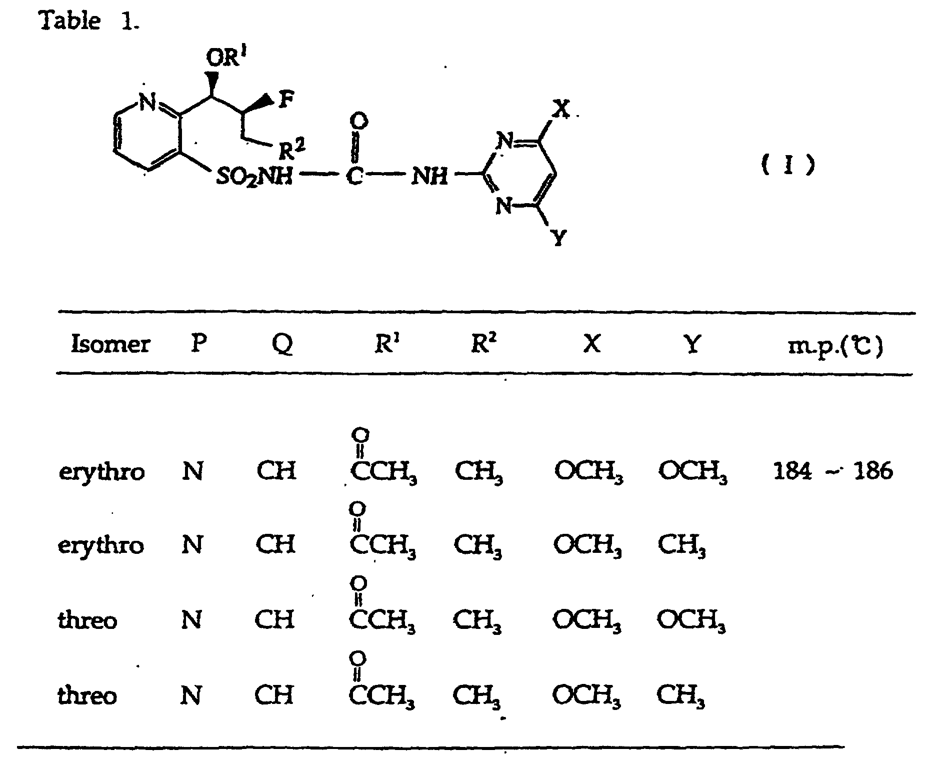

The sulfonamide derivatives having erythro stereochemistry of the above formula(I) according to the present invention are useful as herbicides. The applied method is given below.

### [Utility]

The compounds according to the present invention represent very high activity as pre- or post-emergence herbicides and water surface treatment or leaf treatment herbicides for rice.

The used amount of compound of the present invention is decided by several factor, that is, kinds of weeds, climate or weather, formulations selected, the applied method or the size of weed etc..

The active ingredients can be generally used from 1 g to 1 kg per hectare. Smaller quantity may be used in soil containing low organic matter or sandy soil, young plant or when the herbicidal effect is need of short-termed duration.

The compounds according to the present invention are especially effective as ingredient for control of weed in rice and wheat field, especially leaf-width weed, graminaceae weed and annual or perennial weed. The compounds are particularly effective for control of barnyard grass.

The list of weeds controllable by the compounds of the present invention is given below.

### [the list of weeds]

dicotyledon weeds genus:
   Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Arbutilon, Emex, Datura, Viola, Galeopsis, Papaver, Centaurea.
monocotyledon weeds genus:
   Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Dactyloctenium, Agrostis, Alopecurus, Apera, Heteranthera, Leptochloa.

The compounds of the present invention can be used as alone or in combination with two, three or four additives with other herbicides. The appropriate herbicides for mixed-using with the compounds of the present invention are given bleow. It is particularly useful for control of weeds to use the mixture of the compounds of the present invention and the below herbicides.

| Common Name | |
|---|---|
| acetochlor | acifluorfen |
| AC 252,214 | AC 263,499 |
| acrolein | alachlor |
| ametryn | amitrole |
| AMS | asulam |
| assure | atrazine |
| BAS-514 | barban |
| benefin | bensulfuron methyl |
| bensulide | bentazon |
| benzofluor | benzoylprop |
| bifenox | bromacil |
| bromoxynil | butachlor |
| buthidazole | butralin |
| butylate | cacodylic acid |
| CDAA | CDEC |
| CGA 82725 | CH-83 |
| chloramben | chlorbromuron |
| chlorimuron ethyl | chloroxuron |
| chlorporpham | chlorsulfuron |
| chlortoluron | cinmethylin |
| clethodim | clomazone |
| cloproxydim | clopyralid |
| CMA | cyanazine |
| cycloate | cycluron |
| cyperquat | cyprazine |
| cyprazole | cypromid |
| dalapon | dazomet |
| DCPA | desmediphan |
| desmetryn | diallate |
| dicamba | dichlorbenil |
| dichlorprop | dichlofop |
| diethatyl | difenzoquat |
| dinitramine | dinoseb |
| diphenamid | dipropetryn |
| diquat | diuron |
| DNOC | DOWCO 453 ME |
| DPX-M6316 | DSMA |
| endothall | EPTC |
| ethalfluralin | ethofumesate |
| express | fenac |
| fenoxapropethyl | fenuron |
| fenuron TCA | flamprop |
| fluazifop | fluazifopbutyl |
| fluazifop-P | fluchloralin |
| fluometuron | fluorochloridone |
| fluorodifen | fluoroglycofen |
| fluridone | fomesafen |
| fosamine | glyphosate |
| haloxyfop | harmoney |
| hexaflurate | hexazinone |
| HW-52 | imazamethabenz |
| imazapyr | imazaquin |
| imazethapyr | ioxynil |
| isopropalin | isoproturon |
| isouron | isoxaben |
| karbutilate | lactofen |
| lenacil | linuron |
| MAA | MAMA |
| MCPA | MCPB |
| mecoprop | mefluidide |
| methalpropalin | methabenzthiazuron |
| metham | methazole |
| methoxuron | metolachlor |
| metribuzin | metsulfuron methyl |
| MH | molinate |
| monolinuron | monuron |
| monuron TCA | MSMA |
| My-93 | napropamide |
| naproanilide | naptalam |
| neburon | nitralin |
| nitrofen | nitrofluorfen |
| norea | norfrurazon |
| NTN-801 | oryzalin |
| oxadiazon | oxyfluorfen |
| paraquat | pebulate |
| pendimethalin | perfluidone |
| phenmedipham | picloram |
| PPG-1013 | pretilachlor |
| procyazine | profluralin |
| prometon | prometryn |
| pronamide | propachlor |
| propanil | propazine |
| propham | prosulfalin |
| prynachlor | pyrazon |
| pyrazolate | quizalofop |
| quizalofop ethyl | SC-2957 |
| secbumeton | sethoxydim |
| siduron | simazine |
| SL-49 | sulfometuron methyl |
| TCA | tebuthiuron |
| terbacil | terbuchlor |
| terbuthylazine | terbutol |
| terbutryn | thiameturon methyl |
| thiobencarb | triallate |
| triclopyr | tridiphane |
| trifluralin | trimeturon |
| 2,4-D | 2,4-DB |
| vernolate | X-52 |
| xylachlor | Saturn |
| KH-218 | NSK-850 |
| Pyrazoxyfen | Dimension |
| CH-900 | Mefenacet |
| TSH-888 | Dymron |
| Dimepiperate | Isoxapyrifos |
| Phenobenzuron | JC-940 |
| Esprocab | Methylbencab |
| Phenopylate | Benfuresate |
| S-275 | Quinclorac |
| Londax | NC-311 |
| TH-913 | HW-52 |
| DEH-112 | SKH-301 |
| Bromobutide | BAS517H |
| RE45601 | RE36290 |
| RO173664 | HOE075032 |
| ICIA6051 | DPX^{a}7881 |
| MW80 | CGA136872 |
| DPXV9360 | DPXE9636 |
| SL950 | ICIA02957 |
| CGAI42464 | MY15 |
| MON7200 | WL95481 |
| DPXY6202 | MON15100 |
| SL160 | ICIA0224 |
| LS83556 | BAS518H |
| CGA131036 | DPXL5300 |
| HOE70542 | ICIA0604 |
| ICIA0574 | LS846215 |

### [ Formulation ]

Formulations for the use of the compounds of formula(I) can be prepared in conventional ways. They include dusts, granules, pellets, solutions, suspensions, emulsions, wettable powders, emulsifiable concentrates and the like. Many of these may be applied directly.

Sprayable formulations can be prepared in suitable media and used at spray volumes of from a few liters to several houndred liters per hectare. High strength compositions are primarily used as intermediates for further formulation. The formulations, broadly, contain about 0.1% to 98.9% by weight of active ingredient(s) and at least one of (1) about 0.1% to 20% surfactant(s) and (2) about 1% to 99.8% solid or liquid inert diluent(s) are recommended. More specially, the formulations will contain these ingredients in the following approximate proportions:

**Table 2.**

| Formulations | Weight Percent(%) | | |
|---|---|---|---|
| | Active Ingredient | Diluent Surface | Active Agent |
| Wettable Powders | 20~90 | 1~74 | 1~10 |
| Oil Suspension, Emulsions, Solution | 3~50 | 40~95 | 0.1~15 |
| Emulsifiable Concentrates | | | |
| Aqueous Suspension | 10~50 | 40~84 | 1~20 |
| Dusts | 1~25 | 70~98.9 | 0.1~5 |
| Granules and Pellets | 0.1~95 | 5~99.8 | 0.1~15 |
| High strength Composition | 90~98.9 | 1~10 | 0.1~2 |

Lower or higher levels of active ingredient can, of course, be present depending on the intended use and the physical properties of the compound. Higher ratios of surface active agent to active ingredient are sometimes desirable, and are achieved by incorporation into the formulation or by tank mixing.

Typical solid diluents are mentioned in the writings of Watkins, et al.("Handbook of Insecticide Dust Diluents and Carrier" 2nd Ed., Dorland Books, Caldwell, N.J.,) and other solid diluents can be used.

The more absorptive diluents are preferred for wettable powders and the denser ones for dusts.

Typical liquid diluents and solvents are mentioned in the writings of Marsden ("Solvents Guide", 2nd Ed., Interscience, New York, 1950).

Solubility under 0.1% is preferred for concentrated suspension; concentrated solution is preferably stable against phase separation at 0°C.

The surface active agents and their using method is mentioned in the writings of McCutcheon (McCutcheon's Detergents and Emulsifiers Annual, Mc Publishing Corp., Ridgewood, N.J.,) and Sisely et al. (Sisely snd Wood, "Encyclopedia of Surface Active Agents", Chemical Publishing Co., Inc., New York, 1964).

All the above formulations may contain a small amount of additives to reduce foaming, caking, corrosion and the growth of microorganisms.

The preparation methods of such compositions are well known. A solution can be made only by blending properties and a fine solid composition by blending and pulverizing.

Suspension agents can be made by wet milling method (U.S. Patent No. 3,060,084) and granules and pellets can be made by spraying the active ingredient on preformed granular carrier, or by Agglomeration method (J.E. Browing, "Agglomeration" Chemical Engineering, Dec. 4,1967, pp147 / "Perry's Chemical Engineer's Handbook," 5th Ed., Mcgraw-Hill, New York, 1973, pp 8-57ff).

For further information regarding the art of formulations, see for example: US patent No. 3,235,361 / 3,309,192 / 2,891,855, G. C. Klingman, "Weed Control as a Science", John Wiley and Sons, Inc., New York, 1961, pp.81-96 / J. D. Fryer and S. A. Evans, "Weed Control Handbook", 5th Ed., Blackwell Scientific Publications Oxford, 1968, pp.101~103.

The compounds of the present invention can be used independently and may be used in combination with any other commercial herbicides. To specify some more the manufacturing and using of the compounds of the present invention, the detailed examples are described below.

### EXAMPLE 1

### Erythro-2-(1-acetoxy-2-fluoro-n-butyl)-N-(1,1-dimethylethyl)-3-pyridinesulfonamide

To an erythro-*N*-(1,1-dimethylethyl)-2-(2-fluoro-1-hydroxy-*n*-butyl)-3-pyridinesulfonamide (1.0 g) dissolved in 20 *ml* of methylene chloride were added acetic anhydride (0.37g), pyridine(0.29 g) and *N,N*-dimethyl aminopyridine(50 mg). And the reaction mixture was stirred for 2 hours at room temperature. After the reaction was completed the reaction mixture was diluted with water, acidified with 5% hydrochloric acid solution, and extracted with methylene chloride. The separated organic layer was washed with sodium bicarbonate solution and water(× 2), dried with magnesium sulfate, filtered and concentrated to afford 1.1 g of the desired product.
- ¹H NMR(200MHz, CDCl₃) :: δ 0.93(t, 3H), 1.3(s, 9H), 1.6 ~ 1.9(m, 2H), 2.8(s, 3H), 4.6 ~ 5.0(m, 1H), 5.6(bs, 1H), 6.54 ~ 6.61(dd, 1H), 7.3 ~ 7.4(m, 1H), 8.2 ~ 8.3(m, 1H), 8.65 ~ 8.75(m, 1H).

### EXAMPLE 2

### Erythro-2-(1-acetoxy-2-fluoro-n-butyl)-3-pyridinesulfonamide

An Erythro-2-(1-acetoxy-2-fluoro-*n*-butyl)-*N*-(1,1-dimethylethyl)-3-pyridinesulfonamide was dissolved in 20 *ml* of trifluoroacetic acid. And the reaction mixture was stirred for overnight at room temperature. The reaction mixture was concentrated under the reduced pressure, the residue was washed with sodium bicarbonate solution, dried with magnesium sulfate, filtered and concentrated. The obtained residue was treated with ethyl acetate/n-hexane to afford 0.5 g of the desired product.
- ¹H NMR(200MHz, CDCl₃) :: δ 1.06(t, 3H), 1.6 ~ 2.1(m, 2H), 2.13(s, 3H), 4.7 ~ 5.1(m, 1H), 5.65(br, 1H), 6.61(t, 1H), 7.4 ~ 7.5(m, 1H), 8.4 ~ 8.5(m, 1H), 8.8 ~ 8.9(m, 1H).

### EXAMPLE 3

### Erythro-2-(1-acetoxy-2-fluoro-n-butyl)-N-[(4,6-dimethoxypyrimidin-2-yl) aminocarbonyl]-3-pyridinesulfonamide [ Compound No. 1 ]

To an erythro-2-(1-acetoxy-2-fluoro-*n*-butyl)-3-pyridinesulfonamide (0.5 g) dissolved in acetonitrile(20 *ml*) was added phenyl N-(4,6-dimethoxypyrimidin-2-yl) carbamate at room temperature. To the reaction mixture was added 1,8 - diazabicyclo[5.4.0] undec-7-ene(herein after, "DBU" ; 0.29 g). And the reaction mixture was stirred for 2 hours at room temperature, diluted with methylene chloride, acidified with 5% hydrochloric acid solution, and extracted with methylene chloride. The separated organic layer was washed with water(×2), dried with magnesium sulfate, filtered and concentrated. The obtained residure was treated using ethyl ether to afford 0.6 g of the desired product(white solid).
m.p.: 184 ~ 186 °C
- ¹H NMR(200MHz, CDCl₃) :: δ 0.98(t, 3H), 1.6 ~ 2.0(m, 2H), 2.04(s, 3H), 3.99(s, 6H), 4.7 ~ 5.1(m, 1H), 5.8(s, 1H), 6.6 ~ 6.7(dd, 1H), 7.3(br, 1H), 7.45 ~ 7.55(m, 1H), 8.6 ~ 8.7(m, 1H), 8.8 ~ 8.9(m, 1H).

### Comparative Example 1

### Erythro-2-(1-acetoxy-2-fluoro-n-butyl)-N-[(4,6-dimethoxypyrimidin-2-yl) aminocarbonyl]benzenesulfonamide [ Compound No. 2 ]

To an erythro-2-(1-acetoxy-2-fluoro-*n*-butyl)benzenesulfonamide(2 g) dissolved in acetonitrile(20 *ml*) was added phenyl N-(4,6-dimethoxypyrimidin-2-yl) carbamate. To the reaction mixture was added DBU(1 *ml*). The reaction mixture was stirred for 30 minutes at room temperature, diluted with methylene chloride(100 *ml*) and acidified with 5% hydrochloric acid solution(50 *ml*). The separated organic layer was washed with water(× 2), dried with magnesium sulfate, filtered and concentrated. The obtained residue was treated with ethyl acetate/n-hexane/ethyl ether to afford 26 g of the desired product(white solid).
m.p.: 172 ~ 174 °C
- ¹H NMR(200MHz, CDCl₃) :: δ 0.94(t, 3H, J=8Hz), 1.54 ~ 1.80(m, 2H), 2.00(s, 3H), 3.99(s, 6H), 4.66 ~ 4.93(m, 1H), 5.76(s, 1H), 6.74(dd, 1H, J₁=14.8Hz, J₂= 3Hz), 7.14(brs, 1H), 7.49 ~ 7.62(m, 3H), 8.34 ~ 8.35(m, 1H), 13.06(brs, 1H).
IR(KBr) : ν (C=O) 1710,1755 cm⁻¹

### Comparative Example 2

### Erythro-N-[(4,6-dimethoxypyrimidin-2-yl)aminocarbonyl]-2-(2-fluoro -1-hydroxy-n-butyl)benzenesulfonamide [Compound No.3]

To an erythro-2-(1-acetoxy-2-fluoro-*n*-butyl)-*N*-[(4,6-dimethoxypyrimidin-2-yl)aminocarbonyl]benzenesulfonamide(2 g) dissolved in tetrahydrofuran (60 *ml*) were added lithium hydroxide(0.8 g) and water(10 *ml*) at room temperature. After stirring for 12 hours the reaction mixture was acidified with 5% hydrochloric acid solution at 0 °C and diluted with ethyl acetate(100 *ml*). The separated organic layer was washed with water, dried with magnesium sulfate, filtered and concentrated. The obtained residue was treated with ethyl ether/n-hexane to afford 1.7 g of the desired product(solid).
m.p.: 132 ∼ 134 °C
- ¹H NMR(200MHz, CDCl₃) :: δ 0.95(t, 3H, J=8Hz), 1.57 ~ 1.87(m, 2H), 3.86 ~ 3.92(brs, 1H), 3.96(s, 6H), 4.58 ~ 4.90(m, 1H), 5.76(s, 1H), 5.79 ~ 6.00(m, 1H), 7.27 ~ 8.16(m, 5H), 12.83(brs, 1H).
IR(KBr) : ν (C=O) 1710 cm⁻¹

### EXAMPLE 7

The herbicidal effect of the compounds of the present invention was tested by the greenhouse test, the method is as followings.

### Pre-emergence test

To produce a suitable preparation of active compound, 1 part by weight of active compound was mixed with 5 parts by weight of acetone, 1 part by weight of alkylaryl polyglycol ether as emulsifier was added and the solution diluted with water to the desired concentration. Seeds of the test plants are shown in normal soil and, after 24 hours, watered with the preparation of the active compound.

It is expedient to keep constant the amount of water per unit area.

The concentration of the active compound in the preparation is of no importance, only the amount of active compound applied per unit area being decisive. After three weeks, the degree of damage to the plants was rated in % damage in comparison to the development of the untreated control.
The figures denote
0% = no action (like untreated control)
20% = slight effect
70% = herbicidal effect
100% = total destruction.

In this test, the active compounds(I) according to the preparation examples exhibited a better herbicidal activity against mono- and dicotyledon weeds.

### EXAMPLE 8

### post-emergence test

To produce a suitable preparation of active compound, 1 part by weight of active compound was mixed with 5 parts by weight of acetone, 1 part by weight of emulsifier was added and the solution diluted with water to the desired concentration.

Test plants which had a height of 5∼15 cm were sprayed with the preparation of the active compound in such a way as to apply the particular amounts of active compound desired per unit area. The concentration of the spray liquid was so chosen that the particular amounts of active compound desired were applied in 2,000 *l* of water / ha. After three weeks, the degree of damage to the plants was rated in % damage in comparision to the development of the untreated control.
The figures denote :
0% = no action(like untreated control)
20% = slight effect
70% = herbicidal effect
100% = total destruction.

In this test, the active compounds(I) according to the preparation examples exhibited a better herbicidal activity against mono- and dicotyledon weeds.

### EXAMPLE 9

### Fresh-water treatment paddy submerged test

A plastic pot having a surface area of 60cm² or 140cm² was filled with a small amount of fertilizer, after then, the sterilized paddy soil of puddled state at the depth of 5 cm.

Seeds of barnyard grass, umbrella plant, dayflower, monochoria, toothcup, smartweed, and bulrush et al. and perennial nutrition body of flat-sedge and arrowhead et al., were seeded or planted in surface layer of soil, and pregerminated rice with 2~3 leaves was transplanted one root per pot at the depth of 2 cm.

After planting, the pot was watered for a day at the depth of 2 cm and the manufactured herbicide was spot-treated on the plant in manner sililar to the field condition (4 mg/pot).
Two weeks after treatment, herbicidal effect was measured by the same survey standard as that for field condition.

It is understood that the above examples are illustrative but not limitative of the present invention and that other embodiments within the spirit and scope of the invention will suggest themselves to those skilled in the art.

The following Table 3 represents pre- and post-emergence herbicidal effect of active ingredients.

**Table 3.**

| PRIMARY SCREENING (PADDY SUBMERGED)-Herbicide | | | | | | | | |
|---|---|---|---|---|---|---|---|---|
| Compound No. | DAT* | kg/ha | ORYSA⁽¹⁾ (3 Leafs) | ECHOR⁽¹⁾ | SCPJU⁽²⁾ | MOOVA⁽³⁾ | CYPSE⁽⁴⁾ | SAGPY⁽⁵⁾ |
| 1 | 2 | 0.1 | 0 | 100 | 100 | 100 | 100 | 90 |
| | | 0.025 | 0 | 100 | 100 | 100 | 100 | 90 |
| 2 | 2 | 0.05 | 0 | 90 | 100 | 90 | 100 | 100 |
| | | 0.0125 | 0 | 30 | 100 | 90 | 100 | 100 |
| 3 | 2 | 0.05 | 0 | 90 | 100 | 100 | 100 | 100 |
| | | 0.0125 | 0 | 60 | 100 | 80 | 100 | 100 |

| | | | | | | | | |
|---|---|---|---|---|---|---|---|---|
| (note) *DAT : Day After Treatment | | | | | | | | |
| (1) ORYSA : *Oryza sativa* cv- Dongjin : Rice | | | | | | | | |
| (2) ECHOR : *Echinochloa crus-gall* BEAUV, var. oryzicolo OHWI. : Barnyard *grass* | | | | | | | | |
| (2) SCPJU: *Scirpus juncoides* ROXB. : Bulrush | | | | | | | | |
| (3) CYPSE : *Cyperus serotinus* ROTTB.: Flat-sedge | | | | | | | | |
| (4) MOOVA: *Monochoria vaginalis* PRESL. : Monochoria | | | | | | | | |
| (5) SAGPY: *Sagittaria pygmaea* MIQ. : Arrow head | | | | | | | | |

## Claims

1. A compound of the formula (I) having erythro stereochemistry, 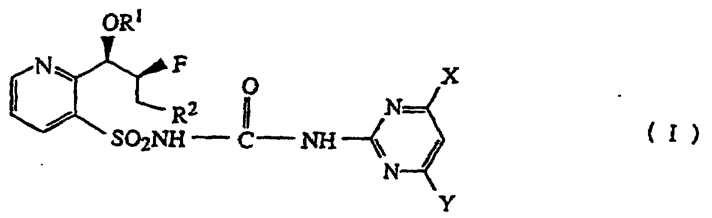 wherein,
R¹ is 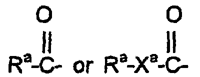 group, wherein R^{a} is C₁-C₃ alkyl, C₁-C₃ haloalkyl, C₂-C₃ alkenyl or C₂-C₃ alkynyl group, wherein X^{a} is O, S, NH or NR^{a} group;
R² is C₁-C₂ alkyl group; and
X and Y are independently halogen atom, C₁-C₂ alkyl, C₁-C₂ alkoxy or C₁-C₂ haloalkoxy group.

2. A compound as defined In claim 1, wherein said R¹ is acetyl group, and said X and Y are respectively methoxy group.

3. A compound as defined in claim 1, wherein said formula (I) is erythro-2-(1-acetoxy-2-fluoro-n-butyl)-N-[(4,6-dimethoxypyrimidin-2-yl)-aminocarbonyl]-3-pyridinesulfonamide.

4. An intermediate compound of formula (II) having erythro stereochemistry, 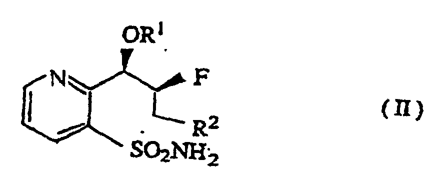 wherein, R¹ and R² are respectively as defined in the above claim 1.

5. An intermediate compound as defined in claim 4, wherein said formula (II) is erythro-2-(1-acetoxy-2-fluoro-n-butyl)-3-pyridinesulfonamide.

6. A herbicidal composition comprising one or more compounds as defined in any of the above claims 1 to 3 as an effective component.

## Patentansprüche

1. Verbindung der Formel (I), welche Erythrostereochemie aufweist, 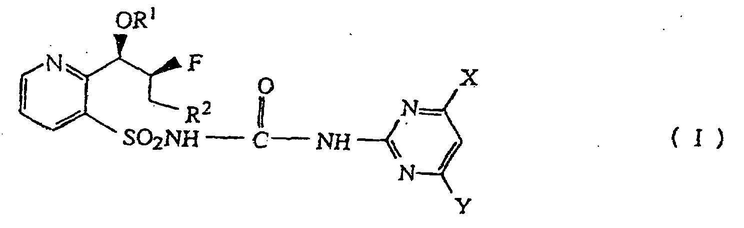 worin,
R¹ eine Gruppe 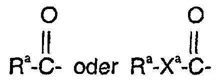 ist, worin R^{a} eine C₁-C₃-Alkyl-, C₁-C₃-Haloalkyl-, C₂-C₃-Alkenyl- oder C₂-C₃-Alkinyl-Gruppe und worin X^{a} O, S, NH oder eine NR^{a}-Gruppe ist,
R² eine C₁-C₂-Alkyl-Gruppe ist und
X und Y unabhängig voneinander Halogenatom, eine C₁-C₂-Alkyl-, C₁-C₂-Alkoxy- oder C₁-C₂-Haloalkoxy-Gruppe sind.

2. Verbindung nach Anspruch 1, worin R¹ eine Acetylgruppe ist und X und Y jeweils Methoxygruppen sind.

3. Verbindung nach Anspruch 1, worin Formel (I) erythro-2-(1-Acetoxy-2-fluoro-n-butyl)-N-[(4,6-dimethoxypyrimidin-2-yl)-aminocarbonyl]-3-pyridinsulfonamid ist.

4. Zwischenverbindung der Formel (II), welche Erythrostereochemie aufweist, 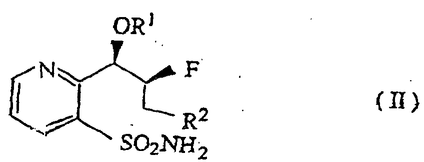 worin R¹ und R² jeweils wie in Anspruch 1 definiert sind.

5. Zwischenverbindung nach Anspruch 4, worin Formel (II) erythro-2-(1-Acetoxy-2-fluoro-n-butyl)-3-pyridinsulfonamid ist.

6. Herbizide Zusammensetzung, welche eine oder mehrere Verbindungen nach einem der vorangegangenen Ansprüche 1 bis 3 als einen wirksamen Bestandteil enthält.

## Revendications

1. Composé de formule (I) ayant une stéréochimie de type érythro, 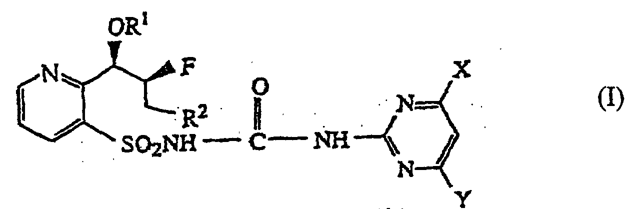 dans laquelle
R¹ est un groupe 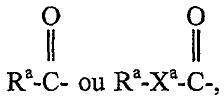 où R^{a} est un groupe alkyle en C₁-C₃, halogénoalkyle en C₁-C₃, alcényle en C₂-C₃ ou alcynyle en C₂-C₃, où X^{a} est O, S, NH ou NR^{a} ;
R² est un groupe alkyle en C₁-C₂ ; et
X et Y représentent chacun indépendamment de l'autre un atome d'halogène, un groupe alkyle en C₁-C₂, alcoxy en C₁-C₂ ou halogénoalcoxy en C₁-C₂.

2. Composé selon la revendication 1, dans lequel ledit radical R¹ est un groupe acétyle, et lesdits radicaux X et Y représentent chacun un groupe méthoxy.

3. Composé selon la revendication 1, dans lequel ladite formule (I) correspond à l'érythro-2-(1-acétoxy-2-fluoro-n-butyl)-N-[(4,6-diméthoxypyrimidine-2-yl)aminocarbonyl]-3-pyridinesulfonamide.

4. Composé intermédiaire de formule (II) ayant une stéréochimie de type érythro, 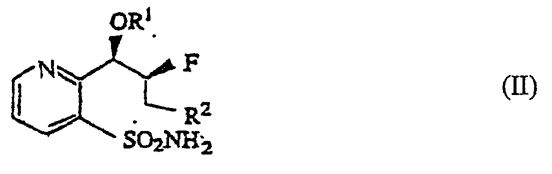 dans laquelle R¹ et R² ont chacun les définitions données dans la revendication 1 ci-dessus.

5. Composé intermédiaire selon la revendication 4, dans lequel ladite formule (II) correspond à l'érythro-2-(1-acétoxy-2-fluoro-n-butyl)-3-pyridinesulfonamide.

6. Composition herbicide comprenant en tant que matière active un ou plusieurs composés selon l'une quelconque des revendications 1 à 3 ci-dessus.
